# EUROPEAN PATENT SPECIFICATION

(11) **EP 4 426 769 B1**
(45) Date of publication and mention of the grant of the patent: **19.03.2025**
(21) Application number: 22813175.1
(22) Date of filing: 28.10.2022
(51) Int. Cl.: C08G 63/08

(54) **A TRIBLOCK COPOLYMER, A PROCESS FOR OBTAINING THEREOF AND USES THEREOF**
TRIBLOCK-COPOLYMER, VERFAHREN ZU DESSEN HERSTELLUNG UND DESSEN VERWENDUNGEN
COPOLYMÈRE TRIBLOC, SON PROCÉDÉ D'OBTENTION ET SES UTILISATIONS

(30) Priority: 05.11.2021 EP 21383008
(43) Date of publication of application: 11.09.2024
(73) Proprietor: Artificial Nature S.L., 08028 Barcelona (ES)
(72) Inventor: MARTINEZ CUTILLAS, Alfredo, 08028 Barcelona (ES); OH, Sejin, 08028 Barcelona (ES); MARTÍNEZ DE ILARDUYA, Antxon, 08028 Barcelona (ES); MARIN RODRÍGUEZ, Xavier, 08028 Barcelona (ES)
(74) Representative: Ponti & Partners, S.L.P
(86) International application number: PCT/EP2022/080298
(87) International publication number: WO 2023/078808

(56) References cited:
- WO-A1-2012/065745
- WO-A1-2014/188340
- MAGLIO G ET AL: "Thermal properties of di- and triblock copolymers of poly(l-lactide) with poly(oxyethylene) or poly(@?-caprolactone)", POLYMER, ELSEVIER, AMSTERDAM, NL, vol. 44, no. 2, 1 January 2003 (2003-01-01), pages 369 - 375, XP004395876, ISSN: 0032-3861, DOI: 10.1016/S0032-3861(02)00764-4
- FUOCO TIZIANA ET AL: "Ring-opening polymerization of [omega]-6-hexadecenlactone by a salicylaldiminato aluminum complex: a route to semicrystalline and functional poly(ester)s", vol. 6, no. 10, 1 January 2015 (2015-01-01), pages 1727 - 1740, XP055911379, ISSN: 1759-9954, Retrieved from the Internet <URL:https://pubs.rsc.org/en/content/articlepdf/2015/py/c4py01445f> [retrieved on 20220411], DOI: 10.1039/C4PY01445F

## Description

### FIELD OF THE INVENTION

The present invention relates to the technology of polymers. In particular the present invention relates to a triblock copolymer, in particular wherein a functional polymacrolactone (PML) is located on the mid-block and polylactic acid on the side-blocks, a process for obtaining thereof and uses thereof.

### BACKGROUND OF THE INVENTION

Polylactic acid (PLA), a biobased, biocompatible, and biodegradable polyester, is a promising polymer with good properties for diverse applications such as packaging, agriculture, or medicine among others. However, it also has deficiencies that must be improved to really have an impact on these fields. Firstly, the lack of functional groups in PLA really limits its application, especially in advanced fields such as biomedical or electronic. For instance, the functional groups could be used to bind bioactive molecules or drugs and use it as drug delivery system. Another example could be the incorporation of hydrophilic molecules that would increase the biodegradation rate. Thus, the addition of extra functional sites would really improve PLA performance and extend its application scope. Secondly, and regarding the mechanical properties, PLA is a rigid and brittle material. That means it can tolerate moderate forces, but it barely deforms before breaking-down (<10%). These are good properties, but some applications may demand the material could undergo plastic deformation or perform flexible behaviour prior to failure. For instance, the design of biomedical temporary implants would rather involve tough and deformable materials instead of a brittle one which could collapse into small pieces inside the body.

Therefore, providing functional sites and improving mechanical properties has been an essential task in the research and development of advanced PLAs. In fact, multiple solutions have been proposed in the literature to modify PLA properties.

Several strategies to provide new functional groups to PLA were proposed. In general, these strategies are based on the use of a functional co-monomer as initiator or on the reaction of functional molecules with PLA end-chain groups. The former approach, as mentioned, involves the use of functional monomers or initiators with the target characteristics. For instance, PEG has been used as initiator in the lactide polymerization to obtain hydrophilic scaffolds of PLA with enhanced degradation rate [1]. The other strategies comprise the grafting of hydrophilic or bioactive molecules into PLA, frequently at the surface, by the end-chain groups. For instance, PLA has been grafted with amine-terminated architectures [2] or with osteoinductive growth factors [3] to improve its hydrophilic and biomedical performance.

Similarly, modification of PLA mechanical properties has already been addressed. A well-known strategy is the synthesis of thermoplastic elastomers (TPEs), which typically have a triblock structure. This solution, owing to the physical crosslinking induced by separated block microphases, combines the rubbery behaviour of soft polymers, the mechanical strength of the hard segments, and the easy processability of thermoplastics. In the literature, there are several examples of PLA copolymerized with softer and more flexible polyesters. Some examples are poly(butylene succinate) (PBS) [4], poly(butylene adipate terephthalate) (PBAT) [5] and lactones such as poly(ε-caprolactone) (PCL) [6], poly(δ-valerolactone) [7] or poly(ε-decalactone) [8]. However, many of these solutions involve high crystalline polymers with saturated long backbones which may difficult the polymer biodegradation. In addition, neither PLA nor these copolymers have more than two functionalities, both located at the end-chain. This is a great limitation in case a further modification is required. For instance, depending on the final application, it might be required to increase the degradation rate or even to add new functional-molecules (bioactive, conductive, barrier...).

In view of above, the problem to be solved by the present invention is to provide a biobased and biocompatible synthetic polymer in the form of a triblock copolymer which has multiple functionalization sites, thus polymers with tunable biodegradation behaviour and mechanical properties can be prepared. Owing to the wide functionalization possibilities, this polymer could be directed towards many application fields such as biomedical, packaging, electronic or agriculture among others.

The inventors have surprisingly found a triblock copolymer which overcomes the previous drawbacks. This triblock copolymer has an ABA structure of a biobased and biodegradable segment A (PLLA or PDLLA) and a biobased, biodegradable, and unsaturated soft segment B (PML). The synergistic effect of combining the functionalization of PLA and the synthesis of TPEs with tunable mechanical properties in a single strategy was surprisingly achieved by using an unsaturated PML as macroinitiator in the lactide ring-opening polymerization (ROP). Moreover, the synthesis pathway and the processing methods for obtaining the triblock copolymer of the invention are cost-effective and easily transferable to the industrial scale.

### SUMMARY OF THE INVENTION

In a first aspect the present invention relates to a triblock copolymer comprising or consisting of the following structure (I):

Polylactic acid (PLA) - X - polylactic acid (PLA) (I)

wherein X is an unsaturated polymacrolactone (PML) or a copolymer containing the PML and a polyester.

In a second aspect, the present invention relates to a process for obtaining a triblock copolymer according to the first aspect of the invention.

In a third aspect, the present invention relates to the use of the triblock copolymer according to the first aspect of the invention.

### BRIEF DESCRIPTION OF THE DRAWINGS

Figure 1 shows the reaction scheme of PMLs obtained by bulk e-ROP of globalide and 6-w-hexadecenlactone.
Figure 2 shows the reaction scheme of PLA-PML-PLA triblock copolymers by bulk ROP using the PMLs as macroinitiators.
Figure 3 shows the ¹H NMR spectrum of the PLLA₁₇₄-PGI₆₃-PLLA₁₇₄ triblock copolymer and peak identification.
Figure 4 shows the ¹³C NMR spectrum of PLLA₁₇₄-PGI₆₃-PLLA₁₇₄ triblock copolymer and the detail of the carbonyl region.
Figure 5 shows the stress-strain curves of PLLA (dark line) and PDLLA₁₀₄-PGI₁₀₆-PLLA₁₀₄ (light line) under tensile load.
Figure 6 shows DSC (a) cooling and (b) heating traces at 10 °C·min⁻¹ of PLLA, PGI and the triblock copolymers.
Figure 7 shows the comparison of ¹H NMR spectra of PDLLA₁₃₉-PGI₈₅-PDLLA₁₃₉ before (light line) and after (dark line) functionalization with COOH-PEG₂₄-COOH.
Figure 8 shows the evolution of a sessile water drop in a rough surface of porous PDLLA₁₃₉-PGI₈₅-PDLLA₁₃₉ and COOH-PEG₂₄-PDLLA₁₃₉-PGI₈₅-PDLLA₁₃₉-PEG₂₄-COOH scaffolds.
Figure 9 shows the stress-strain curves of PDLLA₁₃₉-PGI₈₅-PDLLA₁₃₉ (light line) and COOH-PEG₂₄-PDLLA₁₃₉-PGI₈₅-PDLLA₁₃₉-PEG₂₄-COOH (dark line) under tensile load.

### DETAILED DESCRIPTION OF THE INVENTION

In a first aspect, the present invention relates to a triblock copolymer comprising the following structure (I):

Polylactic acid (PLA) - X - polylactic acid (PLA) (I)

wherein X is an unsaturated polymacrolactone (PML) or a copolymer containing the PML and a polyester.

In a particular embodiment, the present invention relates to a triblock copolymer consisting of the following structure (I):

Polylactic acid (PLA) - X - polylactic acid (PLA) (I)

wherein X is an unsaturated polymacrolactone (PML) or a copolymer containing the PML and a polyester.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. For example, "an unsaturated polymacrolactone" or "the PML" includes the option of one unsaturated polymacrolactone and the option of more than one, identical or different, unsaturated polymacrolactone. Likewise, "a copolymer" includes the option of one copolymer and the option of more than one, identical or different, copolymer, having this copolymer one or more than one, identical or different, PML and one or more than one, identical or different, polyester. Accordingly, there is the option to have a combination of different molecules of PML acting as the PML, as well as a combination of different molecules of polyester acting as the polyester in structure (I). Similarly, the word "or" is intended to include "and" unless the context clearly indicate otherwise.

In a preferred embodiment of the first aspect, the PML is selected from polyglobalide (PGI), poly(ambrettolide) (PAmb), poly(6-ω-hexadecenlactone) (P6HDL) and a combination thereof. In another preferred embodiment of the first aspect, the PML is a combination of any of polyglobalide (PGI), poly(ambrettolide) (PAmb) and poly(6-ω-hexadecenlactone) (P6HDL).

In another preferred embodiment of the first aspect, the polyester is selected from polypentadecalactone (PPDL), polycaprolactone (PCL), polyglycolic acid (PGA), and poly(para-dioxanone). In another preferred embodiment of the first aspect, the polyester is a combination of any of polypentadecalactone (PPDL), polycaprolactone (PCL), polyglycolic acid (PGA) and poly(para-dioxanone).

In another preferred embodiment, "X" in structure (I) is a copolymer consisting of a PML and a polyester.

In another preferred embodiment of the first aspect, the triblock copolymer is end-functionalized or grafted through PML double bonds with molecules containing hydroxyl (-OH), carboxyl (-COOH) or -NH₂ (amine) moieties.

In another preferred embodiment of the first aspect, the triblock copolymer is crosslinked through the self-crosslinking reaction of PML double bonds or through the crosslinking reaction of PML double bonds and other crosslinking molecules such as thiols, amines, maleimides, vinyl sulfones or acrylates.

In a second aspect, the present invention relates to a process for obtaining a triblock copolymer as defined according to the first aspect, i.e. with the following structure (I)

Polylactic acid (PLA) - X - polylactic acid (PLA) (I)

wherein X is an unsaturated polymacrolactone (PML) or a copolymer containing the PML and a polyester,
comprising the steps of:
   a) preparing unsaturated polymacrolactones (PML) by ring-opening polymerization (ROP) of the corresponding unsaturated macrolactones (ML) using a diol as initiator;
wherein in case of X being a block copolymer containing the PML and a polyester, there is an additional step (b-i) or (b-ii) after step (a) and before next step (c) consisting of:
   b-i) when the polyester is derived from cyclic ester monomers, preparing a copolymer containing the PML and a polyester by ring-opening polymerization (ROP) of the cyclic ester monomers using the unsaturated PMLs obtained in step (a) as macroinitiators;
   b-ii) when the polyester is derived from dicarboxylic acids, preparing a copolymer containing the PML and a polyester by polycondensation of dicarboxylic acid or derivatives using the unsaturated PMLs obtained in step (a) as comonomers;
c) polymerizing lactide with a ring-opening polymerization (ROP) using the unsaturated polymacrolactones obtained in step (a) or the copolymers obtained in step (b) as macroinitiators and catalysed by tin (II) 2-ethylhexanoate.

In an alternative embodiment when X in formula (I) is a copolymer, the process for obtaining a triblock copolymer as defined according to the first aspect, i.e. with the following structure (I), comprises the steps of:
1a) preparing a copolymer of PML and a polyester by ring-opening polymerization (ROP) of the corresponding unsaturated macrolactones (ML) and cyclic esters using a diol as initiator; or
1b) preparing a copolymer of PML and a polyester by polycondensation of the corresponding ML, a diol and dicarboxylic acid or derivatives when the comonomers are not cyclic esters;
2) polymerizing lactide with a ring-opening polymerization (ROP) using the unsaturated polymacrolactones obtained in steps (1a) or (1b) as macroinitiators and catalysed by tin (II) 2-ethylhexanoate.

In a further embodiment, after the step of polymerization of the lactide (i.e. step (c) or step (2) depending on the embodiment for the process), there is an additional step consisting of:
- when the triblock copolymer is end-functionalized, reacting hydroxyl (-OH) terminal groups of the triblock copolymer with carboxyl (-COOH) or amine (-NH₂) groups by carbodiimide chemistry; or
- when the triblock copolymer is grafted, reacting the double bond of PML blocks with thiol-containing molecules by thiol-ene "click" chemistry, or
- when the triblock copolymer is crosslinked, reacting the double bonds of PML blocks among themselves or with other crosslinking molecules such as thiols, amines, maleimides, vinyl sulfones or acrylates.

In a preferred embodiment of the second aspect, the PML is selected from polyglobalide (PGI), poly(ambrettolide) (PAmb) and poly(6-ω-hexadecenlactone) (P6HDL). In another preferred embodiment of the second aspect, the PML is a combination of any of polyglobalide (PGI), poly(ambrettolide) (PAmb) and poly(6-ω-hexadecenlactone) (P6HDL).

In another preferred embodiment of the second aspect, the polyester is selected from polypentadecalactone (PPDL), polycaprolactone (PCL), polyglycolic acid (PGA) and poly(para-dioxanone). In another preferred embodiment of the second aspect, the polyester is a combination of any of polypentadecalactone (PPDL), polycaprolactone (PCL), polyglycolic acid (PGA) and poly(para-dioxanone).

In another preferred embodiment, the lactide used in step (c) or step (2) is L-lactide or a mixture of L-lactide and D-lactide.

In another preferred embodiment, the step (a) or steps (1a) or (1b) is carried out in bulk under inert atmosphere.

In another preferred embodiment, the step (a) or steps (1a) or (1b) is carried out with a catalyst. In a further preferred embodiment said catalyst is *Candida Antarctica* Lipase B (CALB) enzyme.

In another preferred embodiment, the step (b-i) or (b-ii) is carried out in bulk under inert atmosphere.

In another preferred embodiment, the step (b-i) or (b-ii) is carried out with a catalyst. In a further preferred embodiment said catalyst is *Candida Antarctica Lipase* B (CALB) enzyme.

In another preferred embodiment, the step (c) or step (2) is carried out in bulk under inert atmosphere in the range of 120-190 °C.

In another preferred embodiment, the concentration of tin (II) 2-ethylhexanoate is in the range of 0.025-0.10% wt.

In another preferred embodiment, the concentration of the macroinitiator PML or the copolymer consisting of PML and a polyester is in the range of 10-50% mol.

In another preferred embodiment, the unreacted monomer is removed and the catalyst is removed or inactivated at the end of each polymerization. There are several ways to carry out such removal or inactivation. In a further preferred embodiment, said removal is carried out by dissolving the reaction mixture in a solvent (for example, chloroform) and precipitating in cold methanol. Nevertheless, when high amounts of reactants are used (for example 1 kg) said removal/inactivation can be carried out without solvents and under vacuum.

In a third aspect, the present invention relates to the use of the triblock copolymer as defined according to the first aspect of the invention in the preparation of:
a) medical devices for tissue regeneration,
b) medical devices for drug delivery systems,
c) lab-on-a-chip and organ-on-a-chip devices,
d) hydrogels for health and smart-agro applications,
e) sensors, biosensors and electrodes,
f) electronic devices,
g) packaging films and trays,
h) textile and synthetic leather,
i) coatings and surface protection solutions, or
j) additives for masterbatches or blend compatibilization.

Block copolymers, particularly with diblock and triblock structures, are frequently used as compatibilizers in immiscible polymer blends. The immiscibility of polymer blends is due to the high interfacial tension between the components, thus the poor interfacial adhesion lead to phase separation and deficient physicochemical properties in comparison to the separated components. Block copolymers play a key role in the compatibilization of immiscible blends because they can be placed at the interface of the separated phases reducing the interfacial tension, thus improving interfacial adhesion. Each block of the copolymer is rather concentrated in one of the separated phases serving as physical bonding between them. As a result, compatibilized blends show improved properties.

Furthermore, this functional triblock copolymers can be directed towards the biomedical field to prepare tissue regeneration scaffolds or drug delivery systems. The control of each block length, the composition and the molecular weight allows the preparation of short-term or long-term medical devices. Moreover, the functionalization of the triblock copolymer by the ending groups or the double bond of PMLs gives the chance of adding hydrophilic and non-fouling molecules such as PEG. The amphiphilic structure of these PEG-containing triblock copolymers would facilitate the encapsulation of drugs, while the non-fouling properties of PEG would reduce the inflammation body response in implantable medical devices. Additionally, drugs or other bioactive molecules could be covalently linked to the triblock copolymer by the unsaturation of PML blocks.

Additionally, PLA has been widely researched as a biobased solution for the packaging industry. However, neat PLA has not the right properties to compete against the current standard polymers such as PET or PE. The mechanical properties, barrier properties and transparency are the main constrains. The triblock copolymer proposed by this invention allow the obtention of flexible materials with good barrier properties, while the transparency could be achieved by functionalization of PML double bonds to decrease the polymer crystallinity.

As mentioned before, the proposed triblock copolymer, apart from having tunable mechanical and physical properties, is also biocompatible. Thus, it offers a competitive advantage to other polymeric substrates traditionally used in the electronic or textile sector. For instance, by adjusting the block length ratio (A/B), our triblock copolymer could meet the flexibility requirements to be used in electronics, where flexibility is an unresolved claim in regard to bio-based and biocompatible polymers. Thereby, it could be used as substrate in domestic electronics or as substrate for the development of sensors and biosensors. Indeed, PLA has shown to provide advanced properties in microfluidic devices such as Organ on a Chips [9], improving the properties of PDMS.

The properties of our triblock copolymer are also compatible within the sectors where fibers are required, such as the textile sector. Moreover, since our triblock copolymer has multiple functionalization sites, it could deliver high added value products for example in sportswear, or in other technical applications related to any biology, human, animal or plant (Smart Agro).

It is noted that any of the embodiments disclosed herein for the product or the process according to the first or second aspect of the invention can be taken alone or combined with any other embodiment disclosed herein unless the context specifies otherwise.

A number of examples will be provided below that are intended to illustrate the invention and in no way limit the scope of the invention, which is established by the attached claims.

### EXAMPLES

### General synthesis procedure

Triblock copolymers were synthetized in two steps. First, PMLs were obtained by enzymatic ring-opening polymerization (e-ROP) of macrolactones (MLs) in bulk, using 1,4-butanediol as initiator. Then, in a second step, PMLs were used as macroinitiators in the lactide bulk ROP catalyzed by tin(ll) 2-ethylhexanoate. Alternatively, random or block copolymers of PMLs and a polyester could be prepared in a single or sequential polymerizations, respectively, by ROP or polycondensation methods. Furthermore, triblock copolymers might be functionalized with hydroxyl (-OH), carboxyl (-COOH) or amine (-NH₂) moieties. Functionalization might be performed to the end-groups using carbodiimide chemistry or to the double bonds of PML blocks by thiol-ene "click" chemistry.

### Polymacrolactones

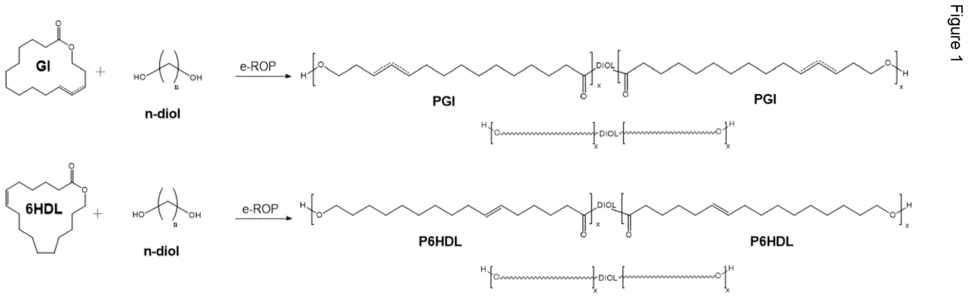

PMLs with molecular weight (*Mₙ*) ranging from 10 to 25 kg·mol⁻¹ were obtained from unsaturated MLs such as globalide (GI) and 6-w-hexadecenlactone (6HDL). Figure 1 shows the synthesis pathway followed to obtain both PMLs. e-ROPs were carried out in bulk under an inert atmosphere. The catalyst was the *Candida antarctica* Lipase B (CALB) enzyme, while 1,4-butanediol was the initiator. The enzyme amount was constant at 5% wt. in every experiment, while the initiator amount was varied depending on the target *Mₙ*. Alternatively, a random copolymer containing PMLs and a polyester could be prepared in a single step by ROP or polycondensation.

### PLA-PML-PLA triblock copolymers

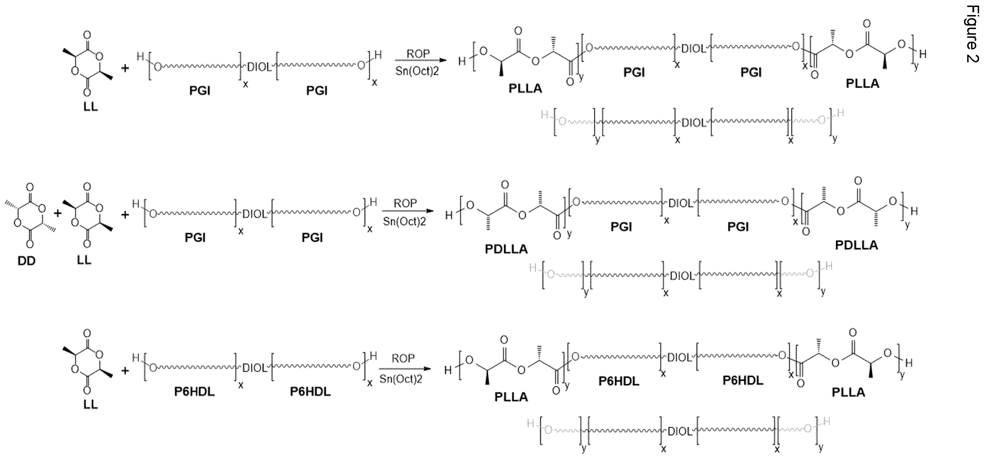

A series of triblock copolymers were synthetized following a single reaction pathway, which is depicted in Figure 2. Either PGI or P6HDL were served as macroinitiators, while L-lactide or the racemic mixture (L-lactide + D-lactide) were used as co-monomers. ROP of lactides was carried out in bulk under an inert atmosphere, at 180 °C, using 0.05% wt. of tin(ll) 2-ethylhexanoate as catalyst. Moreover, the composition of PML and lactide was varied to provide different polymers comprising a wide range of properties. Alternatively, the macroinitiator could be a random or block copolymer consisting of either PGI or P6HDL and a polyester. The random copolymer macroinitiator could be prepared by GI or 6HDL and cyclic esters by ROP or by polycondensation of GI or 6HDL, a diol and dicarboxylic acid derivatives. On the other hand, the block copolymer macroinitiator could be prepared by ROP of cyclic esters using PGI or P6HDL as macroinitiators or by polycondensation of carboxylic acid derivatives using PGI or P6HDL as comonomers. Furthermore, the triblock copolymers could be functionalized by the end-groups using carbodiimide chemistry or by the double bonds using thiol-ene "click" chemistry. Functionalization may comprise the use of hydrophilic or bioactive molecules such as PEG or peptides.

### Purification

At the end of each polymerization, reaction mixture was dissolved in chloroform and precipitated in cold methanol to remove the unreacted monomer and the catalyst. Next, the precipitated was filtered, washed repeatedly with fresh solvent and dried under vacuum for 48 h.

Hereinbelow 3 non-limitative examples of synthesis involving L-lactide, D,L-lactide, globalide and 6-w-hexadecenlactone are described.

### Example 1: Preparation of PLLA₁₀₄-PGI₁₀₆-PLLA₁₀₄ triblock copolymer

The copolymer was prepared in two steps. First, polyglobalide (PGI) was synthetized in a Schlenk tube by enzymatic ring-opening polymerization (e-ROP) at 80 °C in bulk. The tube, provided with a magnetic stirrer, was fluxed with N₂ gas to yield an innert atmosphere. The reactor was heated to 80 °C and a 5% w/w of the enzyme *Candida Antarctica* Lipase B (CALB) with 0.95% mol of 1,4-butanediol were added. After closing the tube with a septum and stopping N₂ gas, the monomer globalide (2 g) was injected to start polymerization. The total reaction time was 5 h. After that time, the reaction product was dissolved in chloroform and filtered to remove enzymes. Filtrated was then precipitated in excess of methanol and washed repeatedly in fresh solvent. Finally, it was dried under vacuum for 48 h at room temperature. The obtained PGI reached a molecular weight of 24.6 kg·mol⁻¹ as determined by ¹H NMR. DSC studies revealed a melting temperature of 48 °C and a crystallization temperature of 32 °C.

PGI was then used as macroinitiator in lactide ROP. A 50 mL 3-necked reactor equipped with a mechanical stirrer was heated to 80 °C and vacuumed at 20 mbar for 15 minutes. Then, aided by N₂ gas to keep the innert atmosphere, PGI (2.5 g) and L-lactide (1.5 g) were supplied to the reactor. Vacuum at 50 mbar was applied again for 15 minutes. Pressure was returned to 1 bar and temperature was increased to 180 °C. At that temperature, 0.05% w/w of stannous octoate respect to lactide were added to start the copolymerization. The total reaction time was 2 h. The resulting polymer was dissolved in chloroform and precipitated in excess of methanol. Precipitated copolymer was dried under vacuum for 48 h. The triblock formation was confirmed by ¹H NMR. It reached a number average molecular weight (*Mₙ*) of 34.2 kg·mol⁻¹ and a weight average molecular weight (*M_{w}*) of 66.5 kg·mol⁻¹as determined by GPC. The copolymer presented two melting peaks at 46.0 °C (PGI block) and 159.8 °C (PLLA blocks) by DSC. PGI block crystallized at 27.4 and -7.2 °C, while PLLA blocks could not crystallize from the melting. The copolymer displayed an elastomeric behaviour at tensile tests reaching 250% elongation at failure, with an elastic modulus of 158 MPa and maximal tensile strength of 4.58 MPa.

### Example 2: Preparation of PDLLA₁₃₉-PGI₈₅-PLLA₁₃₉ triblock copolymer

The copolymer was prepared in two steps. First, polyglobalide (PGI) was synthetized in a Schlenk tube by enzymatic ring-opening polymerization (e-ROP) at 80 °C in bulk. The tube, provided with a magnetic stirrer, was fluxed with N₂ gas to yield an innert atmosphere. The reactor was heated to 80 °C and a 5% w/w of the enzyme *Candida Antarctica* Lipase B (CALB) with 1.18% mol of 1,4-butanediol were added. After closing the tube with a septum and stopping N₂ gas, the monomer globalide (2 g) was injected to start polymerization. The total reaction time was 5 h. After that time, the reaction product was dissolved in chloroform and filtered to remove enzymes. Filtrated was then precipitated in excess of methanol and washed repeatedly in fresh solvent. Finally, it was dried under vacuum for 48 h at room temperature. The obtained PGI reached a molecular weight of 21.5 kg·mol⁻¹ as determined by ¹H NMR.

PGI was then used as macroinitiator in lactide ROP. A 50 mL 3-necked reactor equipped with a mechanical stirrer was heated to 80 °C and vacuumed at 20 mbar for 15 minutes. Then, aided by N₂ gas to keep the innert atmosphere, PGI (2.0 g) and D,L-lactide (2.0 g) were supplied to the reactor. Vacuum at 50 mbar was applied again for 15 minutes. Pressure was returned to 1 bar and temperature was increased to 180 °C. At that temperature, 0.05% w/w of stannous octoate respect to lactide were added to start the copolymerization. The total reaction time was 2 h. The resulting polymer was dissolved in chloroform and precipitated in excess of methanol. Precipitated copolymer was dried under vacuum for 48 h. The triblock formation was confirmed by ¹H NMR. It reached a number average molecular weight (*Mₙ*) of 37.0 kg·mol⁻¹ and a weight average molecular weight (*M_{w}*) of 65.4 kg·mol⁻¹as determined by GPC. The copolymer presented only one melting peak at 41.0 °C due to PGI block, which crystallized in two steps at 23.1 °C and -3.9 °C. PDLLA blocks were amorphous. The copolymer displayed an elastomeric behaviour at tensile tests reaching 230% elongation at failure, with an elastic modulus of 553 MPa and maximal tensile strength of 11.9 MPa.

### Example 3: Preparation of PLLA₂₀₈-P6HDL₄₂-PLLA₂₀₈ triblock copolymer

The copolymer was prepared in two steps. First, polyglobalide (PGI) was synthetized in a Schlenk tube by enzymatic ring-opening polymerization (e-ROP) at 80 °C in bulk. The tube, provided with a magnetic stirrer, was fluxed with N₂ gas to yield an innert atmosphere. The reactor was heated to 80 °C and a 5% w/w of the enzyme *Candida Antarctica* Lipase B (CALB) with 2.36% mol of 1,4-butanediol were added. After closing the tube with a septum and stopping N₂ gas, the monomer 6-w-hexadecenlactone (2 g) was injected to start polymerization. The total reaction time was 5 h. After that time, the reaction product was dissolved in chloroform and filtered to remove enzymes. Filtrated was then precipitated in excess of methanol and washed repeatedly in fresh solvent. Finally, it was dried under vacuum for 48 h at room temperature. The obtained P6HDL reached a molecular weight of 10.5 kg·mol⁻¹ as determined by ¹H NMR.

P6HDL was then used as macroinitiator in lactide ROP. A 50 mL 3-necked reactor equipped with a mechanical stirrer was heated to 80 °C and vacuumed at 20 mbar for 15 minutes. Then, aided by N₂ gas to keep the innert atmosphere, P6HDL (1.0 g) and L-lactide (3.0 g) were supplied to the reactor. Vacuum at 50 mbar was applied again for 15 minutes. Pressure was returned to 1 bar and temperature was increased to 180 °C. At that temperature, 0.05% w/w of stannous octoate respect to lactide were added to start the copolymerization. The total reaction time was 2 h. The resulting polymer was dissolved in chloroform and precipitated in excess of methanol. Precipitated copolymer was dried under vacuum for 48 h. The triblock formation was confirmed by ¹H NMR. It reached a number average molecular weight (*Mₙ*) of 43.0 kg·mol⁻¹ and a weight average molecular weight (*M_{w}*) of 68.0 kg·mol⁻¹as determined by GPC. The copolymer presented two melting peaks at 44.5 °C (P6HDL block) and 1636. °C (PLLA blocks) by DSC. PGI block crystallized at -10.4 °C, while PLLA blocks did it at 99.8 °C. The copolymer displayed an elastomeric behaviour at tensile tests reaching 34% elongation at failure, with an elastic modulus of 491 MPa and maximal tensile strength of 25.5 MPa.

### Example 4: Synthesis of COOH-PEG₂₄-PDLLA₁₃₉-PGI₈₅-PDLLA₁₃₉-PEG₂₄-COOH triblock copolymer

PEG-functionalized triblock copolymers were prepared by esterification of the triblock copolymers and carboxyl-terminated PEG (COOH-PEG₂₄-COOH). As an example, the mixture containing the triblock copolymer PDLLA₁₃₉-PGI₈₅-PDLLA₁₃₉ prepared as described in Example 2 (3.00 g, 0.075 mmol) and a 5-fold excess of the carboxyl-terminated PEG (*Mₙ* ≈ 1,000 g·mol⁻¹, 0.75 g, 0.75 mmol) were added to a round flask provided with a magnetic stirrer. The mixture was dissolved with 20 mL of DCM for 30 min. Then, DCC (0.774 g, 3.75 mmol) and DMAP (0.018 g, 0.15 mmol) were added to the solution and reaction was left to proceed for 24 h at room temperature. Then, reaction mixture was poured into an excess of cold methanol. The precipitate was filtrated, washed repeatedly with fresh methanol, washed with cold diethyl ether (-18 °C) to remove methanol, dried under vacuum for 48 h and stored in a desiccator until further use.

The esterification of carboxyl-terminated PEG and the triblock copolymers succeeded a 95% yield as determined by ¹H NMR. The number average and weight average molecular weights were *Mₙ* = 37 kg·mol⁻¹ and *M_{w}* = 65 kg·mol⁻¹, respectively. The contact angle of PEG-functionalized triblock copolymers was reduced from 90° to 86° because of the PEG addition. Additionally, elongation at break increased from 230% to 380% because of PEG, while the ultimate tensile strength and the elastic modulus decreased from 11.9 MPa to 4.4 MPa and from 553 MPa to 140 MPa, respectively.

### Further examples and results

Following the same method as the described in Example 1, Example 2 and Example 3, up to 12 syntheses were performed to validate the triblock copolymer properties. Table 1 shows all the synthetized triblock copolymers grouped by their PLA and PML composition.

**Table 1. Examples of all the triblock copolymer syntheses.**

| **Copolymer** | **¹H NMR PLA (% mol)** | **¹H NMR *Mₙ* (kg·mol⁻¹)** | ***Mₙ* (kg·mol⁻¹)** | ***M_{w}* (kg·mol⁻¹)** | **PDI** |
|---|---|---|---|---|---|
| PLLA_{y}-PGI₂ₓ-PLLA_{y} | | | | | |
| 2x=42, y=208 | 89.7 | 28.7 | 29.2 | 37.5 | 1.3 |
| 2x=63, y=174 | 79.3 | 29.5 | 37.2 | 54.0 | 1.5 |
| 2x=85, y=139 | 68.0 | 31.4 | 38.5 | 60.5 | 1.6 |
| 2x=106, y=104 | 45.4 | 29.6 | 34.2 | 66.5 | 1.9 |

| PDLLA_{y}-PGI₂ₓ-PDLLA_{y} | | | | | |
|---|---|---|---|---|---|
| 2x=42, y=208 | 88.6 | 44.9 | 45.1 | 65.6 | 1.5 |
| 2x=63, y=174 | 81.3 | 47.0 | 44.6 | 71.0 | 1.6 |
| 2x=85, y=139 | 64.1 | 36.7 | 37.0 | 65.4 | 1.8 |
| 2x=106, y=104 | 57.2 | 34.4 | 39.6 | 72.3 | 1.8 |

| PLLA_{y}-P6HDL₂ₓ-PLLA_{y} | | | | | |
|---|---|---|---|---|---|
| 2x=40, y=208 | 90.0 | 36.0 | 43.2 | 68.0 | 1.6 |
| 2x=59, y=174 | 82.6 | 29.8 | 26.2 | 46.5 | 1.8 |
| 2x=79, y=139 | 71.0 | 29.5 | 29.4 | 55.9 | 1.9 |
| 2x=99, y=104 | 61.3 | 31.2 | 26.9 | 60.2 | 2.2 |

In all cases, the conversion of the monomer into polymer overpassed the 90% and the triblock copolymers were recovered at high yields (> 85%). The theoretical molecular weight in an ideal reaction was targeted at 40 kg·mol⁻¹. Moreover, since copolymers with different PLA/PML compositions were planned, the block length of PLA (segment A) and PML (segment B) were adjusted proportionally. Composition and molecular weights of the triblock copolymers were calculated by ¹H NMR and GPC. Table 1 shows that copolymers were obtained with a PLA composition within the 50-90% mol range. Moreover, molecular weights were in the range of 30-40 kg·mol⁻¹. There are samples with slightly lower molecular weights, which were probably due to the presence of undesired hydroxyl or carboxyl initiating species in the reaction system. On the other hand, deviations in PLA composition were attributed to the loss of lactide by reverse sublimation before starting the reaction. That loss, milligrams, was almost constant, but it became more significant when lower lactide contents were used.

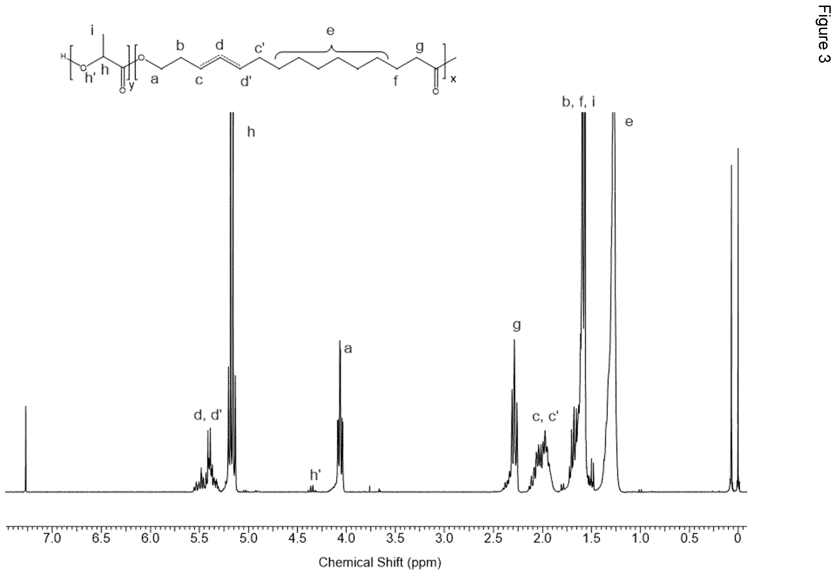

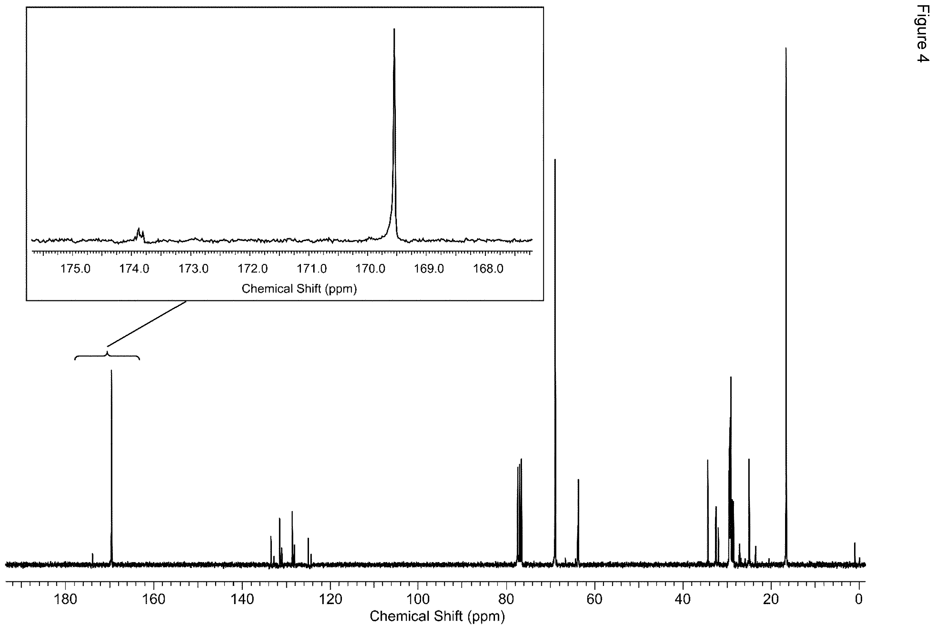

Nevertheless, the triblock structure was confirmed by ¹H and ¹³C NMR. Figure 3 shows the ¹H NMR spectrum of PLLA₁₇₄-PGI₆₃-PLLA₁₇₄. Peak identification confirmed the reaction between PML and lactide, succeeding a triblock structure. Methane (*i*), methyl (*h*) and - (CH₃)CH-OH end-groups (*h*') of PLA blocks were identified at 1.59, 5.17 and 4.35 ppm respectively. The long methylene sequence (*e*), methines (*d, d'*), those methylene around the unsaturation (*c, c'*)*,* those methylene next to the ester group (*a*, *g*)*,* and those next methylene groups (*b*, *f*) were identified at 1.28, 5.40, 2.01, 4.07, 2.29 and 1.69, respectively. Furthermore, ¹³C spectrum depicted in Figure 4 proved neither transesterification nor racemization reactions occurred in the polymerization. PLLA and PML peaks appearing in the carbonyl region (169-175 ppm) were singlets, which means the blocks of each polyester were well defined.

Table 2 shows the mechanical properties of the triblock copolymers. These results evidenced that block length played an important role. The film of neat PLLA, having replicated the molecular weight of the copolymers (40 kg·mol⁻¹), was too brittle even to obtain the specimens for testing. Meanwhile, even those copolymers with only 9-10% mol of PML improved the mechanical performance respect to PLLA. In general, elongation at break (*ε_{b}*) clearly improved with increasing PML, but at the same time involved a reduction on the ultimate tensile strength (*σₘₐₓ*) and the elastic modulus (*E*). Therefore, PLA-based materials with tunable properties ranging from stiff to ductile could be prepared by simply adjusting the PLA/PML composition.

**Table 2. Compositions, molecular weights, and tensile tests of PLA-PML-PLA triblock copolymers.**

| **Copolymer** | **PLA (% mol)** | ***σₘₐₓ* (MPa)** | ***E* (MPa)** | ***ε_{b}* (%)** |
|---|---|---|---|---|
| PLLA | | Too brittle | Too brittle | Too brittle |
| PLLA_{y}-PGI₂ₓ-PLLA_{y} | | | | |
| 2x=42, y=208 | 89.7 | 10.0 ± 3.34 | 951 ± 66.0 | 1.3 ± 0.36 |
| 2x=63, y=174 | 79.3 | 18.3 ± 1.21 | 846 ± 61.1 | 7.1 ± 1.5 |
| 2x=85, y=139 | 68.0 | 10.4 ± 1.46 | 538 ± 43.8 | 39 ± 25 |
| 2x=106, y=104 | 45.4 | 4.58 ± 0.537 | 178 ± 12.0 | 250 ± 27 |

| PDLLA_{y}-PGI₂ₓ-PDLLA_{y} | | | | |
|---|---|---|---|---|
| 2x=42, y=208 | 88.6 | 22.2 ± 4.71 | 1090 ± 113 | 9.2 ± 3.3 |
| 2x=63, y=174 | 81.3 | 21.5 ± 2.13 | 466 ± 42.2 | 200 ± 67 |
| 2x=85, y=139 | 64.1 | 11.8 ± 1.17 | 533 ± 81.2 | 260 ± 94 |
| 2x=106, y=104 | 57.2 | 9.09 ± 1.13 | 381 ± 75.2 | 330 ± 87 |

| PLLA_{y}-P6HDL₂ₓ-PLLA_{y} | | | | |
|---|---|---|---|---|
| 2x=40, y=208 | 90.0 | 25.5 ± 7.82 | 491 ± 234 | 34 ± 33 |
| 2x=59, y=174 | 82.6 | Too brittle | Too brittle | Too brittle |
| 2x=79, y=139 | 71.0 | 11.5 ± 1.46 | 399 ± 179 | 2.7 ± 0.74 |
| 2x=99, y=104 | 61.3 | 11.6 ± 1.83 | 191 ± 84.3 | 3.1 ± 0.90 |

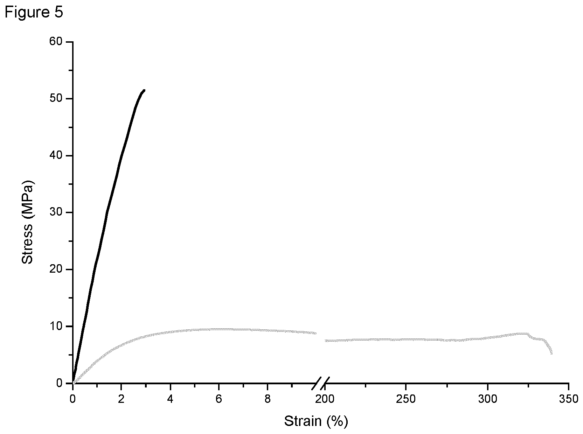

Since the neat PLLA with 40 kg·mol⁻¹ molecular weight was too brittle to be tested, a PLLA with higher molecular weight (*Mₙ* = 72 kg·mol-1, *M_{w}* = 144 kg·mol-1) was synthetized. Figure **5** **¡Error! No se encuentra el origen de la referencia.** shows the results of the tensile strength test performed to high molecular weight PLLA and the triblock copolymer PDLLA₁₀₄-PGI₁₀₆-PDLLA₁₀₄. The triblock structure with the PML in the mid-block displayed a ductile behaviour.

The composition of the triblock structure also influenced the melting temperatures (*T*ₘ) of the copolymers. Table 3 summarizes the thermal properties of the triblock copolymers. As the composition of either PML or PLA increased, the *Tₘ* of the respective block increased as well. Differences on *T*ₘ were about 10 °C. On the contrary, there was not a significant change on the *T*_{g}, degradation onset temperature at 5% (°*T*_{d,5%}) and residual weight (*R_{w}*).

**Table 3. Thermal properties of PLA-PML-PLA triblock copolymers.**

| **Copolymer** | **[PLA]** | **PML** | | | **PLA** | | | | |
|---|---|---|---|---|---|---|---|---|---|
| | **(% mol)** | ***T_{g}* (°C)** | ***Tₘ* (°C)** | ***T_{c}* (°C)** | ***T_{g}* (°C)** | ***Tₘ* (°C)** | ***T_{c}* (°C)** | ***°T_{d,5%}* (°C)** | ***R_{w}* (%)** |
| PLLA_{y}-PGI₂ₓ-PLLA_{y} | | | | | | | | | |
| 2x=42, y=208 | 89.7 | n.d. | 34.2 | -4.9 | 56.8 | 168.0 | 100.3 | 270.6 | 1.47 |
| 2x=63, y=174 | 79.3 | n.d. | 37.7 | -2.2 | 57.2 | 166.8 | 97.7 | 268.5 | 0.28 |
| 2x=85, y=139 | 68.0 | n.d. | 44.5 | -3.9 / 25.6 | 55.8 | 165.3 | n.d. | 275.1 | 1.38 |
| 2x=106, y=104 | 45.4 | n.d. | 46.0 | -7.2 / 27.4 | n.d. | 159.8 | n.d. | 283.3 | 0.23 |

| PDLLA_{y}-PGI₂ₓ-PDLLA_{y} | | | | | | | | | |
|---|---|---|---|---|---|---|---|---|---|
| 2x=40, y=208 | 88.6 | n.d. | 37.0 | -5.6 | 55.2 | - | - | 261.8 | 0.19 |
| 2x=59, y=174 | 81.3 | n.d. | 39.6 | -5.2 / 23.5 | 56.0 | - | - | 260.7 | 0.53 |
| 2x=79, y=139 | 64.1 | n.d. | 41.0 | -3.9 / 23.1 | 50.8 | - | - | 272.4 | 3.51 |
| 2x=99, y=104 | 57.2 | n.d. | 45.9 | -4.5 / 27.4 | n.d. | - | - | 278.7 | 2.04 |

| PLLA_{y}-P6HDL₂ₓ-PLLA_{y} | | | | | | | | | |
|---|---|---|---|---|---|---|---|---|---|
| 2x=40, y=208 | 90.0 | n.d. | 44.5 | -10.4 | 59.2 | 166.3 | 99.8 | 258.3 | 0.51 |
| 2x=59, y=174 | 82.6 | n.d. | 48.1 | 17.1 | n.d. | 158.2 | 91.7 | 270.8 | 0.75 |
| 2x=79, y=139 | 71.0 | n.d. | 53.8 | 33.7 | n.d. | 163.1 | n.d. | 279.5 | 1.28 |
| 2x=99, y=104 | 61.3 | n.d. | 54.5 | 34.8 | n.d. | 162.3 | n.d. | 264.3 | 1.21 |

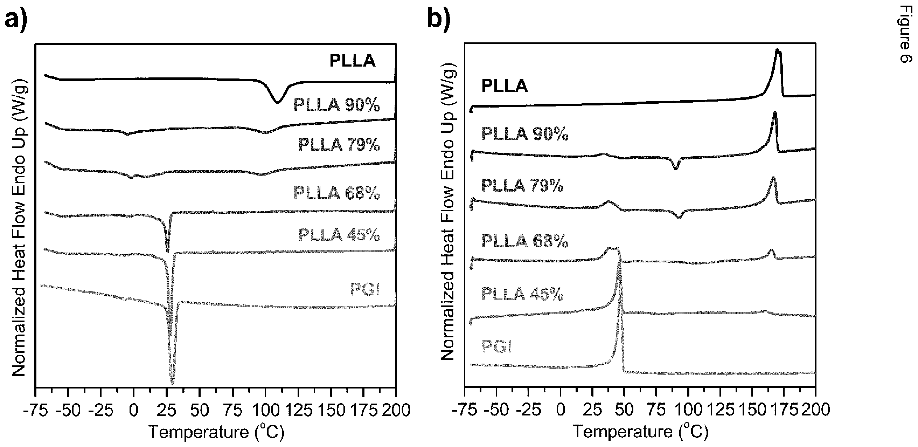

Thermal characterization confirmed the triblock structure as well. Figure 6 shows DSC the first cooling and heating traces at 10 °C·min⁻¹ after removing the thermal history of the sample. Each polyester maintained its thermal behaviour, thus *Tₘ* and *T_{g}* of each block were identified.

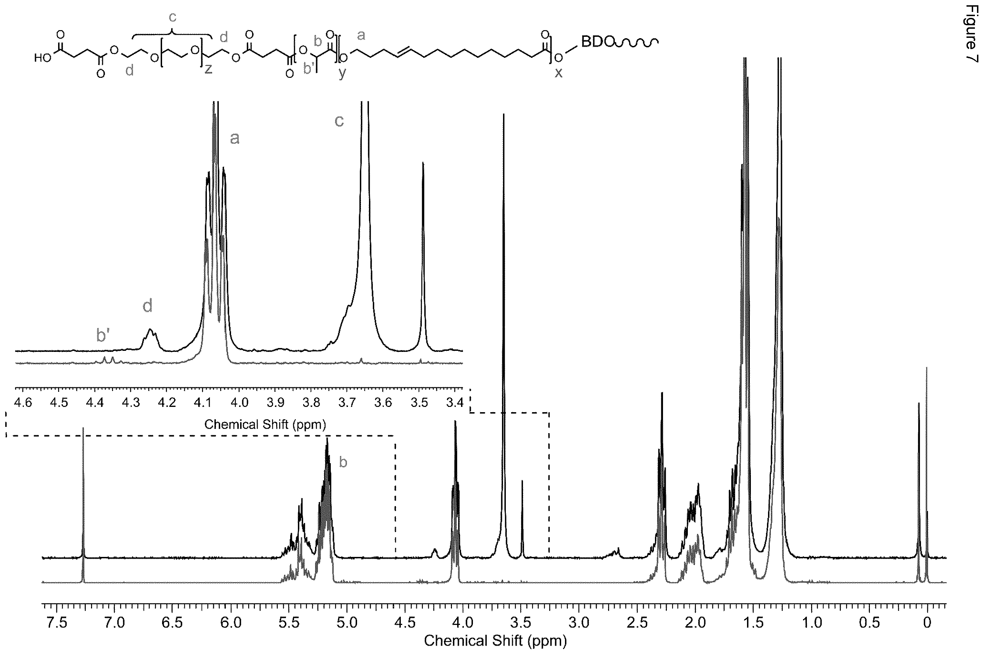

PEG-functionalization of PDLLA₁₃₉-PGI₈₅-PDLLA₁₃₉ was successful. The comparison of¹H NMR spectra depicted in Figure 7 confirmed the esterification of COOH-PEG-COOH and the triblock copolymer. The excess of COOH-PEG₂₄-COOH unreacted was removed by precipitation in methanol and only those PEG chains linked to the triblock copolymer remained. The number of PEG repeating units linked to the triblock copolymer was calculated from the integration of peak (c) in the ¹H NMR spectrum. They matched the number of repeating units indicated by the provider (24) thus virtually complete esterification was accomplished. Furthermore, the number average and weight average molecular weights were *Mₙ* = 37 kg·mol⁻¹ and *M_{w}* = 65 kg·mol⁻¹, respectively. These values were very almost the same as those obtained for the triblock copolymer PDLLA₁₃₉-PGI₈₅-PDLLA₁₃₉. That was reasonable since PEG molecular weight was about 1 kg·mol⁻¹.

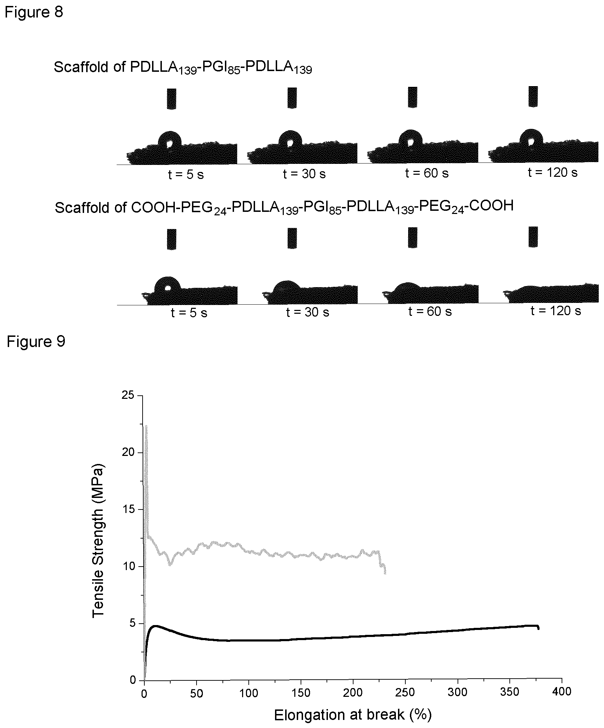

The contact angle of PEG-functionalized triblock copolymers was reduced from 90° to 86° because of the PEG addition. The apparently slight decrease of the contact angle was fundamental to change the surface of triblock copolymers from relatively hydrophobic (>= 90°) to hydrophilic (< 90°). The influence of that small change became more significant when rough surfaces were compared as the roughness intensifies the hydrophobicity or hydrophilicity of flat surfaces. Figure 8 compares the evolution of a water drop in rough surfaces of triblock copolymer scaffolds before and after PEG-functionalization. Contact angle of the relatively hydrophobic triblock copolymer increased from 90° to 114° because of the roughness increase. Moreover, the water drop remained moveless along the 120 seconds of experiment. Conversely, the scaffold of the hydrophilic PEG-functionalized copolymer reduced the contact angle from 86° to 74° in comparison to the flat film. Indeed, the water drop progressively reduced the contact angle along the time to virtually disappear from the scaffold surface.

Regarding the mechanical properties, PEG-functionalized copolymers displayed more ductile and flexible properties in comparison to neat triblock copolymers. Figure 9 shows representative stress-strain curves under tensile load of PDLLA₁₃₉-PGI₈₅-PDLLA₁₃₉ before and after PEG-functionalization. Elongation at break was increased from 230% to 380% due to the plasticization effect of PEG chains. At the same time, the elastic modulus and the ultimate tensile strength were reduced from 553 MPa to 140 MPa and from 11.9 MPa to 4.4 MPa, respectively.

### Characterization

All the synthetized PLA-PML-PLA were analysed using the following techniques:
- Nuclear Magnetic Resonance (NMR). ¹H and ¹³C spectra were obtained using a Bruker AMX-300 at 25 °C, 300.1 and 75.5 MHz frequencies, respectively. 64 scans were done for ¹H spectra, while 1,000-10,000 were done for ¹³C spectra. Polymer samples, 10 mg for ¹H spectra and 50 mg for ¹³C spectra, were dissolved in deuterated chloroform (CDCl₃). As internal reference, tetramethylsilane (TMS) is utilized. Additionally, in some cases, two-dimensional spectroscopy methods such as homonuclear ¹H-¹H (COSY) and heteronuclear correlation ¹H-¹³C (HETCOR) were applied.
- Gel Permeation Chromatography (GPC). Molecular weight was determined by GPC utilizing a Waters equipment. Two columns and two different eluents were used depending on the polymer samples. Eluents were hexafluoroisopropanol (HFIP) and tetrahydrofuran (THF). Molecular weight was calculated by comparison to polymethylmethacrylate (PMMA) in HFIP and polystyrene (PS) in THF standards. Samples were prepared dissolving 1 mg of polymer in 1 mL of solvent. Measurements were performed at 35 °C.
- Differential Scanning Calorimetry (DSC). Thermal transitions were evaluated by calorimetric scans, which were performed on a Perkin-Elmer Pyris 1 and on a DSC 8500. Thermograms were recorded from 4-6 mg of polymer under a continuous 20 mL min⁻¹ nitrogen flux. Standards used for temperature and enthalpy calibration were indium and zinc.
- Thermogravimetric Analysis (TGA). Thermal stability of polymers were studied within the temperature range of 50 and 600 °C. Analyses were performed on a Mettler-Toledo TGA/DSC 1 Star System, using nitrogen flux and with a heating rate of 10 °C min⁻¹.
- Tensile tests. Mechanical testing was carried out in a 500N zwicki equipment from Zwick Roell. Tensile and elongation data were recorded by the testXpert^{®} III software. Tests were performed using 5B standard specimens obtained from polymer films following the standard ISO 527-2.
- Contact angle (CA). Drop shape analysis with sessile drops was performed by OCA 20 (DataPhysics Instruments GmbH, Filderstadt) and SCA20 software. CAs were measured 5 seconds after 0.500 µL of distilled water were dropped in 3x1 cm² film samples at room temperature. Values of the left and right CAs were measured and averaged. At least 10 measurements were performed in 3 different film polymer samples. Dynamic CAs were also carried out by taking pictures of the water drop at scheduled times.

### REFERENCES

1. Zhu, X.; Zhong, T.; Huang, R.; Wan, A. Preparation of Hydrophilic Poly(Lactic Acid) Tissue Engineering Scaffold via (PLA)-(PLA-b-PEG)-(PEG) Solution Casting and Thermal-Induced Surface Structural Transformation. J. Biomater. Sci. Polym. Ed. 2015, 26, 1286-1296.
2. Janorkar, A. V.; Fritz, E.W.; Burg, K.J.L.; Metters, A.T.; Hirt, D.E. Grafting Amine-Terminated Branched Architectures from Poly(L-Lactide) Film Surfaces for Improved Cell Attachment. J. Biomed. Mater. Res. - Part B Appl. Biomater. 2007, 81, 142-152.
3. Edlund, U.; Dånmark, S.; Albertsson, A.C. A Strategy for the Covalent Functionalization of Resorbable Polymers with Heparin and Osteoinductive Growth Factor. Biomacromolecules 2008, 9, 901-905.
4. Jia, L.; Yin, L.; Li, Y.; Li, Q.; Yang, J.; Yu, J.; Shi, T.; Fang, Q.; Cao, A. New Enantiomeric Polylactide-Block-Poly(Butylene Succinate)-Block- Polylactides: Syntheses, Characterization and in Situ Self-Assembly. Macromol. Biosci. 2005, 5, 526-538.
5. Ding, Y.; Lu, B.; Wang, P.; Wang, G.; Ji, J. PLA-PBAT-PLA Tri-Block Copolymers: Effective Compatibilizers for Promotion of the Mechanical and Rheological Properties of PLA / PBAT Blends. Polym. Degrad. Stab. 2018, 147, 41-48.
6. Maglio, G.; Migliozzi, A.; Palumbo, R. Thermal Properties of Di- and Triblock Copolymers of Poly(I-Lactide) with Poly(Oxyethylene) or Poly(ε-Caprolactone). Polymer (Guildf). 2002, 44, 369-375.
7. Jing, Z.; Shi, X.; Zhang, G. Synthesis and Properties of Biodegradable Supramolecular Polymers Based on Polylactide- Block-Poly(δ-Valerolactone)-Block-Polylactide Triblock Copolymers. Polym. Int. 2017, 66, 1487-1497.
8. Olsén, P.; Borke, T.; Odelius, K.; Albertsson, A.-C. ε-Decalactone: A Thermoresilient and Toughening Comonomer to Poly( I -Lactide). Biomacromolecules 2013, 14, 2883-2890.
9. Ongaro, A.E.; Di Giuseppe, D.; Kermanizadeh, A.; Miguelez Crespo, A.; Mencattini, A.; Ghibelli, L.; Mancini, V.; Wlodarczyk, K.L.; Hand, D.P.; Martinelli, E.; et al. Polylactic Is a Sustainable, Low Absorption, Low Autofluorescence Alternative to Other Plastics for Microfluidic and Organ-on-Chip Applications. Anal. Chem. 2020, 92, 6693-6701.

## Claims

1. A triblock copolymer comprising the following structure (I):
Polylactic acid (PLA) - X - polylactic acid (PLA) (I)
wherein X is an unsaturated polymacrolactone (PML) or a copolymer containing the PML and a polyester.

2. A triblock copolymer, according to claim 1, consisting of the following structure (I):
Polylactic acid (PLA) - X - polylactic acid (PLA) (I)
wherein X is an unsaturated polymacrolactone (PML) or a copolymer containing the PML and a polyester.

3. The triblock copolymer according to claim 1 or 2, wherein the PML is selected from polyglobalide (PGI), poly(ambrettolide) (PAmb), poly(6-ω-hexadecenlactone) (P6HDL) and a combination thereof.

4. The triblock copolymer according to any of claims 1 to 3, wherein the polyester is selected from polypentadecalactone (PPDL), polycaprolactone (PCL), polyglycolic acid (PGA), poly(para-dioxanone) and a combination thereof.

5. Process for obtaining a triblock copolymer according to any of claims 1 to 4 with the following structure (I)
Polylactic acid (PLA) - X - polylactic acid (PLA) (I)
wherein X is an unsaturated polymacrolactone (PML) or a copolymer containing the PML and a polyester,
comprising the steps of:
a) preparing unsaturated polymacrolactones (PML) by ring-opening polymerization (ROP) of the corresponding unsaturated macrolactones (ML) using a diol as initiator;
wherein in case of X being a copolymer containing the PML and a polyester, there is an additional step (b) after step (a) and before next step (c) consisting of:
b-i) when the polyester is derived from cyclic ester monomers, preparing a copolymer containing the PML and a polyester by ring-opening polymerization (ROP) of the cyclic ester monomers using the unsaturated PMLs obtained in step (a) as macroinitiators;
b-ii) when the polyester is derived from non-cyclic ester monomers, preparing a copolymer containing the PML and a polyester by polycondensation of non-cyclic ester monomers using the unsaturated PMLs obtained in step (a) as comonomers;
c) polymerizing lactide with a ring-opening polymerization (ROP) using the unsaturated polymacrolactones obtained in step (a) or the copolymer obtained in step (b) as macroinitiators and catalysed by tin (II) 2-ethylhexanoate.

6. Process for obtaining a triblock copolymer according to any of claims 1 to 4 with the following structure (I)
Polylactic acid (PLA) - X - polylactic acid (PLA) (I)
wherein X is a copolymer containing the PML and a polyester,
comprising the steps of:
1a) preparing a copolymer of PML and a polyester by ring-opening polymerization (ROP) of the corresponding unsaturated macrolactones (ML) and cyclic esters using a diol as initiator; or
1b) preparing a copolymer of PML and a polyester by polycondensation of the corresponding ML, a diol and dicarboxylic acid or derivatives when the comonomers are not cyclic esters;
2) polymerizing lactide with a ring-opening polymerization (ROP) using the unsaturated polymacrolactones obtained in steps (1a) or (1b) as macroinitiators and catalysed by tin (II) 2-ethylhexanoate.

7. Process, according to claims 5 or 6, further comprising the step of:
- when the triblock copolymer is end-functionalized, reacting hydroxyl (-OH) terminal groups of the triblock copolymer with carboxyl (-COOH) or amine (-NH₂) groups by carbodiimide chemistry; or
- when the triblock copolymer is grafted, reacting the double bond of PML blocks with thiol-containing molecules by thiol-ene "click" chemistry; or
- when the triblock copolymer is crosslinked, reacting the double bonds of PML blocks among themselves or with other crosslinking molecules.

8. Process, according to any of claims 5 to 7, wherein the PML is selected from polyglobalide (PGI), poly(ambrettolide) (PAmb), poly(6-ω-hexadecenlactone) (P6HDL), and a combination thereof.

9. The process according to any of claims 5 to 8, wherein the polyester is selected from polypentadecalactone (PPDL), polycaprolactone (PCL), polyglycolic acid (PGA), poly(para-dioxanone), and a combination thereof.

10. Process, according to claim 5, step (c) or claim 6, step (2) or any of claims 7 to 9, wherein the lactide is L-lactide or a mixture of L-lactide and D-lactide.

11. Process, according to claim 5, step (a), or claim 6, step (1a) or (1b), or any of claims 7 to 10, which step is carried out with a catalyst.

12. Process, according to claim 5, step (c) or claim 6, step (2), or any of claims 7 to 11, which step is carried out in bulk under inert atmosphere in the range of 120-190 °C.

13. Process, according to any of claims 5 to 12, wherein the concentration of tin (II) 2-ethylhexanoate is in the range of 0.025-0.10% wt.

14. Process, according to any of claims 5 to 13, wherein at the end of each polymerization the unreacted monomer is removed and the catalyst is removed or inactivated.

15. Use of the triblock copolymer according to any of claims 1 to 4, for preparing:
a) medical devices for tissue regeneration,
b) medical devices for drug delivery systems,
c) lab-on-a-chip and organ-on-a-chip devices,
d) hydrogels for health and smart-agro applications,
e) sensors, biosensors and electrodes,
f) electronic devices,
g) packaging films and trays,
h) textile and synthetic leather,
i) coatings and surface protection solutions, or
j) additives for masterbatches or blend compatibilization.

## Patentansprüche

1. Triblockcopolymer, umfassend die folgende Struktur (I):
Polymilchsäure (PLA) - X - polymilchsäure (PLA) (I)
wobei X ein ungesättigtes Polymacrolacton (PML) oder ein das PML und einen Polyester enthaltendes Copolymer bedeutet.

2. Triblockcopolymer nach Anspruch 1, bestehend aus der folgenden Struktur (I):
Polymilchsäure (PLA) - X - polymilchsäure (PLA) (I)
wobei X ein ungesättigtes Polymacrolacton (PML) oder ein das PML und einen Polyester enthaltendes Copolymer bedeutet.

3. Triblockcopolymer nach Anspruch 1 oder 2, wobei das PML ausgewählt ist aus Polyglobalid (PGI), Poly-(ambrettolid) (PAmb), Poly-(6-w-hexadecenlacton) (P6HDL) und einer Kombination davon.

4. Triblockcopolymer nach einem der Ansprüche 1 bis 3, wobei der Polyester ausgewählt ist aus Polypentadecalacton (PPDL), Polycaprolacton (PCL), Polyglycolsäure (PGA), Poly-(para-dioxanon) und einer Kombination davon.

5. Verfahren zur Herstellung eines Triblockcopolymers nach einem der Ansprüche 1 bis 4 mit der folgenden Struktur (I):
Polymilchsäure (PLA) - X - polymilchsäure (PLA) (I)
wobei X ein ungesättigtes Polymacrolacton (PML) oder ein das PML und einen Polyester enthaltendes Copolymer bedeutet,
umfassend die Stufen:
a) Herstellen ungesättigter Polymacrolactone (PML) durch Ringöffnungspolymerisation (ROP) der entsprechenden ungesättigten Macrolactone (ML) unter Verwendung eines Diols als Initiator;
wobei für den Fall, dass X ein das PML und einen Polyester enthaltendes Copolymer bedeutet, eine zusätzliche Stufe (b) nach Stufe (a) und vor nächster Stufe (c) erfolgt, die besteht aus:
b-i) wenn sich der Polyester von cyclischen Estermonomeren ableitet, Herstellen eines das PML und einen Polyester enthaltenden Copolymers durch Ringöffnungspolymerisation (ROP) der cyclischen Estermonomeren unter Verwendung der in Stufe (a) erhaltenen ungesättigten PMLs als Makroinitiatoren;
b-ii) wenn sich der Polyester von nicht-cyclischen Estermonomeren ableitet, Herstellen eines das PML und einen Polyester enthaltenden Copolymers durch Polykondensation von nicht-cyclischen Estermonomeren unter Verwendung der in Stufe (a) erhaltenen ungesättigten PMLs als Comonomere;
c) Polymersieren von Lactid durch Ringöffnungspolymerisation (ROP) unter Verwendung der in Stufe (a) erhaltenen ungesättigten Polymakrolactone oder des in Stufe (b) erhaltenen Copolymers als Makroinitiatoren unter Katalyse durch Zinn(II)-2-ethylhexanoat.

6. Verfahren zur Herstellung eines Triblockcopolymers nach einem der Ansprüche 1 bis 4 mit der folgenden Struktur (I):
Polymilchsäure (PLA) - X - polymilchsäure (PLA) (I)
wobei X ein PML und einen Polyester enthaltendes Copolymer bedeutet,
umfassend die Stufen:
1a) Herstellen eines Copolymers von PML und einem Polyester durch Ringöffnungspolymerisation (ROP) der entsprechenden ungesättigten Makrolactone (ML) und cyclischen Ester unter Verwendung eines Diols als Initiator oder
1b) Herstellen eines Copolymers von PML und einem Polyester durch Polykondensation des entsprechenden ML, eines Diols und einer Dicarbonsäure oder von Derivaten davon, wenn es sich bei den Comonomeren um nichtcyclische Ester handelt;
2) Polymerisieren von Lactid durch Ringöffnungspolymerisation (ROP) unter Verwendung der in den Stufen (1a) oder (1b) erhaltenen ungesättigten Polymacrolactone als Markroinitiatoren unter Katalyse mit Zinn(II)-2-ethylhexanoat.

7. Verfahren nach Ansprüchen 5 oder 6, ferner umfassend die Stufe:
- wenn das Triblockcopolymer endfunktionalisiert ist, Umsetzen von Hydroxyl (-OH) Endgruppen des Triblockcopolymeren mit Carboxyl- (-COOH) oder Amin- (-NH₂) Gruppen durch Carbodiimid-Chemie; oder
- wenn das Triblockcopolymer gepfropft ist, Umsetzen der Doppelbindung von PML-Blöcken mit thiolhaltigen Molekülen durch Thiol-En-"Click"-Chemie; oder
- wenn das Triblockcopolymer vernetzt ist, Umsetzen der Doppelbindungen von PML-Blöcken untereinander oder mit anderen vernetzenden Molekülen.

8. Verfahren nach einem der Ansprüche 5 bis 7, wobei das PML ausgewählt ist aus Polyglobalid (PGI), Poly-(ambrettolid) (PAmb), Poly-(6-w-hexadecenlacton) (P6HDL) und einer Kombination davon.

9. Verfahren nach einem der Ansprüche 5 bis 8, wobei der Polyester ausgewählt ist aus Polypentadecalacton (PPDL), Polycaprolacton (PCL), Polyglycolsäure (PGA), Poly-(para-dioxanon) und einer Kombination davon.

10. Verfahren nach Anspruch 5, Stufe (c) oder Anspruch 6, Stufe (2) oder einem der Ansprühe 7 bis 9, wobei es sich beim Lactid um L-Lactid oder um ein Gemisch aus L-Lactid und D-Lactid handelt.

11. Verfahren nach Anspruch 5, Stufe (a) oder Anspruch 6, Stufe (1a) oder (1b) oder einem der Ansprüche 7 bis 10, wobei die Stufe mit einem Katalysator durchgeführt wird.

12. Verfahren nach Anspruch 5, Stufe (c) oder Anspruch 6, Stufe (2) oder einem der Ansprüche 7 bis 11, wobei die Stufe in Masse unter einer inerten Atmosphäre im Bereich von 120-190 °C durchgeführt wird.

13. Verfahren nach einem der Ansprüche 5 bis 12, wobei die Konzentration von Zinn(II)-2-ethylhexanoat im Bereich von 0,025-0,10 Gew.-% liegt.

14. Verfahren nach einem der Ansprüche 5 bis 13, wobei am Ende einer jeden Polymerisation das nicht umgesetzte Monomer entfernt wird und der Katalysator entfernt oder inaktiviert wird.

15. Verwendung des Triblockcopolymers nach einem der Ansprüche 1 bis 4, zur Herstellung von
a) medizinischen Vorrichtungen zur Geweberegeneration,
b) medizinischen Vorrichtungen für Arzneistoff-Abgabesysteme,
c) Labor-auf-einem-Chip-Vorrichtungen und Organ-auf-einem-Chip-Vorrichtungen,
d) Hydrogelen für Heilanwendungen oder Smart-Agro-Anwendungen,
e) Sensoren, Biosensoren und Elektroden,
f) elektronischen Vorrichtungen,
g) Verpackungsfolien und -schalen,
h) Textilien und Kunstleder,
i) Überzüge und Oberflächenschutz-Lösungen oder
j) Additive für Masterbatches oder Gewährleistung von Mischungsverträglichkeit.

## Revendications

1. Copolymère tribloc comprenant la structure (I) suivante :
acide polylactique (PLA) - X - acide polylactique (PLA) (I)
dans lequel X est une polymacrolactone insaturée (PML) ou un copolymère contenant la PML et un polyester.

2. Copolymère tribloc selon la revendication 1, constitué de la structure (I) suivante :
acide polylactique (PLA) - X - acide polylactique (PLA) (I)
dans lequel X est une polymacrolactone insaturée (PML) ou un copolymère contenant la PML et un polyester.

3. Copolymère tribloc selon la revendication 1 ou 2, dans lequel la PML est choisie parmi polyglobalide (PGI), poly(ambrettolide) (PAmb), poly(6-ω-hexadécenlactone) (P6HDL) et une combinaison de ceux-ci.

4. Copolymère tribloc selon l'une quelconque des revendications 1 à 3, dans lequel le polyester est choisi parmi polypentadécalactone (PPDL), polycaprolactone (PCL), acide polyglycolique (PGA), poly(para-dioxanone) et une combinaison de ceux-ci.

5. Procédé d'obtention d'un copolymère tribloc selon l'une quelconque des revendications 1 à 4 présentant la structure (I) suivante
acide polylactique (PLA) - X - acide polylactique (PLA) (I)
dans lequel X est une polymacrolactone insaturée (PML) ou un copolymère contenant la PML et un polyester,
comprenant les étapes consistant à :
a) préparer des polymacrolactones insaturées (PML) par polymérisation par ouverture de cycle (ROP) des macrolactones insaturées (ML) correspondantes en utilisant un diol comme initiateur ;
dans lequel, dans le cas où X est un copolymère contenant la PML et un polyester, une étape supplémentaire (b) existe après l'étape (a) et avant l'étape (c) suivante, consistant à :
b-i) lorsque le polyester est dérivé de monomères d'ester cyclique, préparer un copolymère contenant la PML et un polyester par polymérisation par ouverture de cycle (ROP) des monomères d'ester cyclique en utilisant les PML insaturées obtenues à l'étape (a) en tant que macro-initiateurs ;
b-ii) lorsque le polyester est dérivé de monomères d'ester non cyclique, préparer un copolymère contenant la PML et un polyester par polycondensation de monomères d'ester non cyclique en utilisant les PML insaturées obtenues à l'étape (a) en tant que comonomères ;
c) polymériser le lactide avec une polymérisation par ouverture de cycle (ROP) en utilisant les polymacrolactones insaturées obtenues à l'étape (a) ou le copolymère obtenu à l'étape (b) en tant que macro-initiateurs et après catalyse par du 2-éthylhexanoate d'étain (II).

6. Procédé d'obtention d'un copolymère tribloc selon l'une quelconque des revendications 1 à 4 présentant la structure (I) suivante
acide polylactique (PLA) - X - acide polylactique (PLA) (I)
dans lequel X est un copolymère contenant la PML et un polyester, comprenant les étapes consistant à :
1a) préparer un copolymère de PML et d'un polyester par polymérisation par ouverture de cycle (ROP) des macrolactones insaturées (ML) et des esters cycliques correspondants en utilisant un diol comme initiateur ; ou
1b) préparer un copolymère de PML et d'un polyester par polycondensation du ML correspondant, d'un diol et d'un acide dicarboxylique ou de dérivés lorsque les comonomères ne sont pas des esters cycliques ;
2) polymériser le lactide avec une polymérisation par ouverture de cycle (ROP) en utilisant les polymacrolactones insaturées obtenues dans les étapes (1a) ou (1b) en tant que macro-initiateurs et après catalyse par du 2-éthylhexanoate d'étain (II).

7. Procédé, selon les revendications 5 ou 6, comprenant en outre l'étape consistant à :
- lorsque le copolymère tribloc est fonctionnalisé en extrémité, faire réagir des groupes terminaux hydroxyle (-OH) du copolymère tribloc avec des groupes carboxyle (-COOH) ou amine (-NH₂) par chimie de carbodiimides ; ou
- lorsque le copolymère tribloc est greffé, faire réagir la double liaison des blocs PML avec des molécules contenant du thiol par chimie « click » thiol-ène ; ou
- lorsque le copolymère tribloc est réticulé, faire réagir les doubles liaisons des blocs PML entre elles ou avec d'autres molécules de réticulation.

8. Procédé selon l'une quelconque des revendications 5 à 7, dans lequel la PML est choisie parmi polyglobalide (PGI), poly(ambrettolide) (PAmb), poly(6-ω-hexadécenlactone) (P6HDL) et une combinaison de ceux-ci.

9. Procédé selon l'une quelconque des revendications 5 à 8, dans lequel le polyester est choisi parmi polypentadécalactone (PPDL), polycaprolactone (PCL), acide polyglycolique (PGA), poly(para-dioxanone) et une combinaison de ceux-ci.

10. Procédé selon la revendication 5, étape (c), ou la revendication 6, étape (2), ou l'une quelconque des revendications 7 à 9, dans lequel le lactide est le L-lactide ou un mélange de L-lactide et de D-lactide.

11. Procédé selon la revendication 5, étape (a), ou la revendication 6, étape (1a) ou (1b), ou l'une quelconque des revendications 7 à 10, laquelle étape est réalisée avec un catalyseur.

12. Procédé selon la revendication 5, étape (c), ou la revendication 6, étape (2), ou l'une quelconque des revendications 7 à 11, laquelle étape est réalisée en vrac sous atmosphère inerte dans la plage de 120 à 190°C.

13. Procédé selon l'une quelconque des revendications 5 à 12, dans lequel la concentration de 2-éthylhexanoate d'étain (II) est dans la plage de 0,025 à 0,10 % en masse.

14. Procédé selon l'une quelconque des revendications 5 à 13, dans lequel à la fin de chaque polymérisation le monomère n'ayant pas réagi est éliminé et le catalyseur est éliminé ou inactivé.

15. Utilisation du copolymère tribloc selon l'une quelconque des revendications 1 à 4, pour préparer :
a) des dispositifs médicaux pour une régénération tissulaire,
b) des dispositifs médicaux pour des systèmes d'administration de médicament,
c) des dispositifs de laboratoire sur puce et d'organe sur puce,
d) des hydrogels pour les applications santé et de type smart-agro,
e) des capteurs, biocapteurs et électrodes,
f) des dispositifs électroniques,
g) des films d'emballage et des plateaux,
h) du textile et du cuir synthétique,
i) des revêtements et des solutions de protection de surface, ou
j) des additifs pour des mélanges maîtres ou une compatibilisation de mélange.
